# EUROPÄISCHE PATENTSCHRIFT

(11) **EP 3 083 931 B1**
(45) Veröffentlichungstag und Bekanntmachung des Hinweises auf die Patenterteilung: **13.02.2019**
(21) Anmeldenummer: 14823903.1
(22) Anmeldetag: 17.12.2014
(51) Int. Cl.: C12M 1/00, C12M 3/06, C12M 3/00, C12M 1/12

(54) **MIKROFLUIDISCHER BIOREAKTOR MIT MODULAREM AUFBAU ZUR SYNTHESE VON ZELLMETABOLITEN, DAS VERFAHREN ZUR NUTZUNG SOWIE DESSEN VERWENDUNG**
MICROFLUIDIC BIOREACTOR WITH MODULAR DESIGN FOR SYNTHESIZING CELL METABOLITES, METHOD FOR USING SAME, AND USE THEREOF
BIORÉACTEUR MICROFLUIDIQUE PRÉSENTANT UNE STRUCTURE MODULAIRE POUR LA SYNTHÈSE DE MÉTABOLITES CELLULAIRES, PROCÉDÉ POUR SON EXPLOITATION ET SON UTILISATION

(30) Priorität: 20.12.2013 DE 102013114634
(43) Veröffentlichungstag der Anmeldung: 26.10.2016
(73) Patentinhaber: Karlsruher Institut für Technologie, 76131 Karlsruhe (DE)
(72) Erfinder: MAISCH, Jan, 76275 Ettlingen (DE); AHRENS, Ralf, 76149 Karlsruhe (DE); SOBICH, Shukhrat, 76187 Karlsruhe (DE); GUBER, Andreas, 76227 Karlsruhe (DE); KREPPENHOFER, Kristina, 81373 München (DE); NICK, Peter, 79104 Freiburg (DE)
(86) Internationale Anmeldenummer: PCT/EP2014/003394
(87) Internationale Veröffentlichungsnummer: WO 2015/090581

(56) Entgegenhaltungen:
- EP-A1- 2 184 344
- EP-A1- 2 525 225
- WO-A1-2012/016711
- WO-A1-2013/011962
- WO-A1-2013/086329
- WO-A2-03/085379
- WO-A2-2007/047772
- WO-A2-2008/025351
- DE-A1-102006 030 068
- US-A1- 2006 154 361
- US-A1- 2006 199 260
- US-A1- 2007 048 727

## Beschreibung

Die vorliegende Erfindung betrifft ein Verfahren zur Gewinnung von Zellmetaboliten mit einem mikrofluidischen Bioreaktor mit modularem Aufbau.

Die Mikrofluidik ist ein wachsendes, dynamisches Forschungsgebiet, weil die Integration mikrofluidischer Strukturen, beispielsweise Kanäle oder Reservoire, in Mikrosysteme für viele technische Anwendungsgebiete interessant ist. Zu diesen Anwendungsgebieten gehören laut Whitesides [1] insbesondere die analytische Chemie, die molekulare Biologie und die Mikroelektronik. Die Anfang der 1990er Jahre ursprünglich für eine hochparallelisierte chemische Analyse entwickelten mikrofluidischen Chips (Kapillarelektrophorese im Chipformat, [2, 3]) fanden schnell in modifizierter Form Anwendungsmöglichkeiten in anderen Wissenschaftsdisziplinen, wie z.B. der Molekularbiologie, und auch in der Industrie. Insbesondere die grundlegenden Arbeiten um Whitesides (z.B. Soft Lithographie, [1, 4]) beschleunigten diese Entwicklungen maßgeblich, da hier einfache und schnelle Methoden entwickelt wurden, die auch in nicht spezialisierten Labors eingesetzt werden konnten, um mikro- und nanofluidische Chips und Strukturen herzustellen, die sich insbesondere für biomedizinische Anwendungen eignen.

Es sind nur sehr wenige mikrofluidische Systeme zur Kultivierung von Zellen pflanzlicher Herkunft bekannt.

Ko et al. [5] beschreiben ein mikrofluidisches System zur Kultivierung von Pflanzenzellen aus PDMS, in dem Protoplasten aus grünen Blättern von Tabak *Nicotiana tabacum* L. über zehn Tage kultiviert wurden. Der vorgestellte Chip weist eine Zellkulturkammer in Form eines Kanals auf, einen Mikrofilter sowie einen Einlass und einen Auslass. Der Mikrofilter ist in der kanalförmigen Zellkammer angeordnet und dient als Rückhaltebarriere für die in der Zellkammer befindlichen Zellen. Der Kanal wird mit Zellkulturmedium mit einer Geschwindigkeit von 50-100 µl/min durchströmt. Die Zellviabilität wurde durch einen fluoreszenten Vitalfarbstoff qualitativ bestätigt, aber nicht quantifiziert. Auf den vorgelegten mikroskopischen Aufnahmen sind zahlreiche abgestorbene Zellen zu sehen.

Thiebaud et al [6] zeigen einen PDMS-Mikrofluidikchip zur Zellkultur tierischer Zellen mit 8 Zellkulturkanälen und 8 Einlassöffnungen, wobei die Zellen hier auf der mit Laminin behandelten PDMS-Kanalinnenseite anhaften und dadurch bei Durchströmung des Kanals mit Zellkulturmedium im Kanal zurückgehalten werden. US2006/0199260 offenbart ein Bioreaktor zum Kultivieren von Zellen, der Sauerstoffdurchlässig ist, um das Zellwachstum zu unterstützen.

US2006/0199260 offenbart auch gestapelte Produktionsabläufe, allerdings produzieren alle Einzel-Batches das gleiche Produkt, das so im Laufe des Stapels aufkonzentriert wird.

EP2184344 A1 offenbart einen Bioreaktor zum Kultivieren von Zellen und zur Analyse der Wirkung von Substanzen und physikalischen Einflüssen auf Zellen.

Ausgehend hiervon besteht die Aufgabe darin, die Einschränkungen und Nachteile des Standes der Technik zu überwinden.

### Folgendes Verfahren wird vorgeschlagen:

Verfahren zur Gewinnung von Zellmetaboliten mit einem mikrofluidischen Bioreaktor (1) umfassend - mindestens zwei Module (2, 3, 4, 5, 6, X) mit jeweils einer Kavität, die mit jeweils einer Membran (20) in jeweils eine Zellkammer (211, 311, 411) für die Aufnahmen von Zellen und jeweils einer mit einer flüssigen Lösung mit mindestens einem Zusatzstoff und Zellmetaboliten durchströmbare Stoffkammer (221, 321, 421) unterteilt ist, wobei die jeweilige Membran einen Reaktionsbereich (10) aufweist, in dem die Membran (20) zumindest teilweise durchlässig für die Lösung mit dem mindestens einem Zusatzstoff und die Zellmetaboliten ist, sowie - ein Fluidleitungssystem (16) für die flüssige Lösung mit dem mindestens einem Zusatzstoff und die Zellmetaboliten, das die Stoffkammer (221, 321, 421) in Reihe und/oder parallel miteinander verbindet - sowie ein Mittel für eine unidirektionale Durchströmbarkeit des Fluidleitungssystem. mit den Verfahrensschritten: a) Einbringen von Zellen in die Zellkammer (211, 311, 411) b) Anlegen eines Flüssigkeitsstroms einer flüssigen Lösung mit mindestens einem Zusatzstoff im Fluidleitungssystem des mikrofluidischen Bioreaktors (1) zur Synthese des mindestens einen Zellmetaboliten, wobei die flüssige Lösung mit dem mindestens einem Zusatzstoff mindestens zwei Module nacheinander durchfliest und dabei jeweils über die Stoffkammern (221, 321, 421) durch die Membran in die Zellkammer (211, 311, 411) eindringt, in der Zellkammer (211, 311, 411) mit den Zellen mindestens ein Produkt synthetisiert und die flüssige Lösung mit dem mindestens einem Zusatzstoff und dem mindestens einem Produkt durch die Membran über die Stoffkammern (221, 321, 421) in das Fluidleitungssystem (16) zurückgeführt wird, wobei das Produkt des zuvor durchflossenen Moduls das Edukt des nachfolgenden Moduls ist, bis das Produkt der Zellmetabolit ist c) Entnahme des mindestens einen Zellmetaboliten aus dem Flüssigkeitsstrom.

Die Unteransprüche beschreiben jeweils vorteilhafte Ausgestaltungen der Erfindung.

Der pflanzliche Sekundärmetabolismus erzeugt zahlreiche Komponenten mit medizinischer Wirksamkeit. Diese werden in der Pflanze nur in spezifischen Zellen gebildet und erfordern die Interaktion verschiedener Gewebe. Daher ist die biotechnologische Erzeugung in batch-Kulturen nicht praktikabel. Die Extraktion aus der Pflanze ist mühsam und dadurch limitiert, dass die Komponenten nur in wenigen Zellen vorliegen. Zudem sind viele dieser Pflanzen bedroht und selten.

Der mikrofluidische Bioreaktor aus mehreren Modulen ist dem Gewebeverbund von Zellen nachempfunden, in denen verschiedene Zelltypen in Kompartimenten nebeneinander existieren und miteinander kommunizieren. Die Produkte jeder Zelllinie werden der nächsten Zelllinie als Edukte zur Verfügung gestellt. Die Zellen der Zelllinie im jeweils letzten Modul erzeugen aus den Produkten der Zellen der vorgeschalteten Zelllinien das gewünschte Endprodukt, den Zellmetaboliten gelöst in einer Flüssigkeit. Diese Kopplung erfolgt modular - jedes Modul beinhaltet Zellen einer Zelllinie, bei denen bevorzugt ein spezifischer metabolischer Schritt durch Überexpression des entsprechenden Schlüsselenzyms hochreguliert ist. Das Produkt wird dann über einen Exporter aus der Zelle hinausgeschleust und dann in das nächste Modul des mikrofluidischen Bioreaktors transportiert, wo es als Substrat für das nächste Modul dient. Diese Module können modular rekombiniert werden, so dass mit einer geringen Zahl von Modulen zahlreiche metabolische Verzweigungen möglich sind. Die einzelnen Zelllinien werden in separaten flachen Zellkammern untergebracht, die über eine poröse Membran mit darunter liegenden Stoffkammern verbunden sind, durch die die Versorgung und der Stoffaustausch sichergestellt werden. Dabei hat jede Zellkammer ein Befüllsystem, um sie mit den Zellen der jeweiligen Zelllinie zu befüllen. Jede einzelne Zellkammer bildet zusammen mit der dazugehörigen Stoffkammer ein Modul und diese Module können dann beliebig miteinander verbunden werden. Dadurch lassen sich im Durchfluss wertvolle Komponenten, die im natürlichen pflanzlichen System limitiert sind und aufgrund ihrer chemischen Komplexität nicht abiotisch erzeugt werden können, herstellen. Durch das modulare Prinzip lassen sich schon aus wenigen Bauelementen zahlreiche Varianten erzeugen - auch solche, die in der Natur noch gar nicht vorkommen.

Die Bezeichnungen "Zellen" im gesamten Text umfasst neben natürlichen und transgenen Zellen tierischer oder pflanzlicher Zelllinien auch Protoplasten, d.h. Zellen, denen die Zellwand durch enzymatischen Verdau entfernt wurde, Hefen, Pilze und Bakterien.

Der mikrofluidische Bioreaktor umfasst mindestens zwei Module, deren Reihenfolge frei wählbar ist. Jedes Modul umfasst jeweils eine Kavität, die mit jeweils einer Membran in jeweils eine Zellkammer für die Aufnahmen von Zellen und jeweils einer mit einer flüssigen Lösung mit mindestens einem Zusatzstoff und Zellmetaboliden durchströmbare Stoffkammer unterteilt ist. Die Zellkammer dient zur Aufnahme von natürlichen oder transgenen Zellen, insbesondere natürlicher oder transgener pflanzlicher Zellen oder Protoplasten. Die Zellkammer und ihr Befüllsystem bilden eine erste Einheit eines jeden Moduls des mikrofluidischen Bioreaktors. Die Stoffkammern sind durch ein Fluidleitungssystem in Reihe und/oder parallel miteinander verbunden. Die flüssige Lösung mit dem mindestens einem Zusatzstoff und den Zellmetaboliten bildet einen Fluidikkreislauf, der die Stoffkammern unidirektional durchströmt. Die Stoffkammer und das dazugehörige Fluidleitungssystem bilden eine zweite Einheit des mikrofluidischen Bioreaktors.

Die jeweilige Membran weist einen Reaktionsbereich auf, indem die Membran zumindest teilweise für die Lösung mit dem mindestens einem Zusatzstoff und die Zellmetaboliten durchlässig ist und es ermöglicht, dass der mindestens eine Zusatzstoff in der flüssigen Lösung der Reaktionseinheit mit den Zellen in der Zellkammer in Kontakt kommt, d.h. es besteht zumindest im Reaktionsbereich der Membran zumindest teilweise die Möglichkeit des stofflichen Austausches zwischen der Zell-und der Stoffkammer.

Stofflicher Austausch bedeutet, dass neben Wasser auch organische Moleküle und Salze, sogenannte Zusatzstoffe, entweder durch Diffusion oder über natürliche oder künstliche Transportsysteme über den Fluidikkreislauf aus der Stoffkammer in die Zellkammer und/oder umgekehrt gelangen. Durch den stofflichen Austausch gelangen die Zusatzstoffe von der Stoffkammer in die Zellkammer, wobei Reaktanden immer in beide Richtungen wandern können, d.h. von der Zellkammer in die Stoffkammer und von der Stoffkammer in die Zellkammer. In anderen Ausgestaltungen ermöglicht der Reaktionsbereich der Membran den stofflichen Austausch mehrerer oder aller Zusatzstoffe in beide Richtungen.

Die unidirektional durchströmbaren Stoffkammern der mindestens zwei Module stehen über das Fluidleitungssystem miteinander in Flüssigkeitsverbindung, d.h. die Stoffkammer des ersten Moduls ist über das Fluidleitungssystem so mit der Stoffkammern des zweiten Moduls und den Stoffkammern eventuell vorhandener weiterer Module gekoppelt, dass die Flüssigkeit zuerst die Stoffkammer des ersten Moduls und anschließend, gemäß der Reihenfolge, die Stoffkammern des mindestens einem weiteren Moduls durchströmt.

Der Fluidikkreislauf der Stoffkammern der mindestens zwei Module ist mit einer druckerzeugenden Vorrichtung, vorzugsweise einer Pumpe, insbesondere Peristaltik- oder Spritzenpumpen, oder einer Absaugeeinheit so verbunden, dass die Flüssigkeit nacheinander die Stoffkammern der mindestens zwei hintereinander geschalteten Module durchströmt. Der Fluidikkreislauf der Stoffkammer des ersten Moduls des Bioreaktors wird durch eine Flüssigkeit in einem Vorratsgefäß oder eine kontinuierlich die Flüssigkeit mischende Anlage gespeist. In einer weiteren Ausgestaltung wird der Druckgradient über unterschiedliche Höhenlage des Vorratsgefäßes im Vergleich zum Modul erzeugt. Die Flussrate lässt sich durch den angelegten Druck beeinflussen und hängt von der Reaktionsgeschwindigkeit der einzelnen Syntheseschritte und der Aufnahme der Reaktanden in die Zellen der verwendeten Zelllinien ab. Die Synthese wird bei einer Flussrate von 1-1000 µl/min, bevorzugt bei 10-500µl/min und besonders bevorzugt bei 25-150 µl/min durchgeführt.

In einer Ausgestaltung erfolgt die Kopplung der Module linear bzw. in Reihe, d.h. ein Modul ist hinter ein weiteres Modul geschaltet und der Fluidikkreislauf durchläuft alle Module. In einer weiteren Ausgestaltung erfolgt die Kopplung parallel, d.h. hinter ein Modul werden mindestens 2 voneinander unabhängige Module gekoppelt. Dabei wird der Fluidikkreislauf in mehrere Ströme aufgetrennt. Jeder Strom durchläuft einen Strang dieser parallel-geschalteten Module. Die Anzahl der parallel-geschalteten Module je Strom ist voneinander unabhängig. Dadurch ist es möglich, aus einer Vorstufe mehrere Produkte zeitgleich herzustellen. In einer besonderen Ausgestaltung erfolgt die Kopplung linear und parallel, d.h. hinter ein Modul werden mehr als ein voneinander unabhängige Module geschaltet, wobei hinter diese ein weiteres Modul geschaltet wird, dass durch beide vorhergehenden, voneinander unabhängigen Module versorgt wird. Dabei wird der Fluidikkreislauf nach einem Modul in mehrere Ströme aufgetrennt, welche dann die parallel-geschalteten Module durchlaufen. Nach dem Durchlaufen der parallel-geschalteten Module werden die mehreren Ströme des Fluidikkreislaufs vereinigt und durchlaufen gemeinsam das mindestens eine nachgeschalteten Modul. Damit ist es z.B. möglich, aus einem ersten Produkt, das als Edukt in die mehreren parallelgeschalteten Module gelangt, mehrere voneinander verschiedene Produkte zu gewinnen. Diese mehreren voneinander verschiedenen Produkte werden dann als Edukte in mindestens ein gemeinsames Modul geleitet und dort von den Zellen in ein Produkt umgewandelt. Das ist vor allem dann vorteilhaft, wenn die Produkte der betreffenden Module die Ausbeute des jeweils anderen Produkts nachhaltig stören, d.h. wenn also ein Reaktand A zum Produkt B und ein Reaktand C zum Produkt D umgesetzt werden soll, jedoch bei Anwesenheit von B die Synthese von D nicht oder nur unzureichend stattfindet. Die Anzahl der parallel-geschalteten Module pro Strom ist voneinander unabhängig und wird durch die Syntheseroute bedingt. Auch die Anzahl der Verzweigungspunkte linear - parallel wird durch die Syntheseroute bestimmt. So ist es auch möglich mehrere voneinander unabhängige Verzweigungspunkte in einem mikrofluidischen Bioreaktor zu etablieren.

Die verwendeten Zusatzstoffe sind ausgewählt unter Nährstoffen, Wachstumsregulatoren, Immunabwehrstoffen, Aktivatoren, Hemmstoffen und Elicitoren, ausgewählt unter HrpZ, flg22, Resveratrol, oder induzierenden oder selektiven Agentien.

Die Nährstoffe, ausgewählt unter organischen Molekülen, Aminosäuren, Fetten, Salzen, Kohlenhydraten, Vitamine, Makro-, Mikro- oder Spurenelementen werden dazu eingesetzt, die Zellen in den einzelnen Zellkammern zu ernähren.

Es werden je nach verwendeter Zellkultur alle in der Literatur bekannten pflanzlichen Kulturmedien verwendet, der Fachmann wählt das jeweilige Kulturmedium abhängig von Zelllinie, durchzuführender Synthese und etwaiger gentechnologischer Veränderung der Zelllinie aus. Neben den Standardmedien werden auch auf spezielle Zellkulturen angepasste Nährmedien [7] eingesetzt.

Unter Reaktand versteht man die Ausgangsstoffe bzw. Edukte für den jeweiligen Syntheseschritt im jeweiligen Modul. Die Produkte jeder Zelllinie in einem Modul werden als Reaktand der nächsten Zelllinie im nachgeschalteten Modul zur Verfügung gestellt. Die letzte Zelllinie produziert das gewünschte Produkt, den gewünschten Zellmetabolit. Die flüssige Lösung mit mindestens einem Zusatzstoff des Fluidikkreislaufs umfasst daher mindestens einen Reaktanden, um die Synthese zu starten. In einer weiteren Ausgestaltung ist der Reaktand ein Nährstoff oder ein Aktivator.

Als Aktivatoren und Hemmstoffe finden literaturbekannte pflanzliche Hormone, Wachstumsregulatoren und andere bioaktive Moleküle aber auch Temperatur- und Lichtsignale bzw. elektrische Signale Einsatz [8].

Aktivatoren, ausgewählt unter pflanzlichen Hormonen, Wachstumsregulatoren und anderen bioaktiven Moleküle, wie Indolessigsäure, 1-Napthylessigsäure, 2,4-Dichlorphenoxyessigsäure, Jasmonsäure oder Abscisinsäure, werden verwendet, um bestimmte Reaktionen in den einzelnen Zelllinien auszulösen. Dazu wird der Aktivator in einer Ausgestaltung direkt in den Fluidikkreislauf gegeben, d.h. der Aktivator durchströmt alle Stoffkammer der verschiedenen Module. Je nach verwendeter Membran wandern die Aktivatoren dann in die Zellkammern der einzelnen Module und kommen mit den Zellen in Kontakt. Die Auswahl der Membranen und der Zelllinien in den einzelnen Modulen hängt also von der Wirkung der Aktivatoren auf die Zelllinien ab. In einer weiteren Ausgestaltung wird der Aktivator direkt z.B. über die Versorgungsleitung zu der jeweiligen Zelllinie gegeben, d.h. nur die Zellen in diesem Modul kommen mit dem Aktivator in Kontakt. Sind hinter dieses Modul weitere Module geschaltet, besteht die Möglichkeit, dass nachfolgende Zellen mit dem Überschuss des Aktivators in der Lösung des Fluidikkreislaufs in Kontakt kommen, wenn dieser die Membran passieren kann. In einer bevorzugten Ausgestaltung ist die Membran für den Aktivator nicht permeabel, d.h. er kann sie nicht passieren und verbleibt in der Zellkammer des Moduls zu dem er hinzugefügt wurde.

Hemmstoffe, ausgewählt unter pflanzlichen Hormonen, Wachstumsregulatoren und anderen bioaktiven Moleküle, wie 1-N-Naphthylphthalamsäure, Oryzalin, Latrunculin oder Phalloidin, und Temperatur- und Lichtsignale, werden verwendet, um bestimmte Reaktionen in den einzelnen Zelllinien zu unterdrücken. Dazu wird der Hemmstoff entweder direkt in den Fluidikkreislauf gegeben, d.h. der Hemmstoff durchströmt alle Stoffkammer der verschiedenen Module. Je nach verwendeter Membran wandern die Hemmstoffe dann in die Zellkammern der einzelnen Module und kommen mit den Zellen in Kontakt. Die Auswahl der Membranen und der Zelllinien in den einzelnen Modulen hängt also von der Wirkung der Hemmstoffe auf die Zelllinien ab. In einer weiteren Ausgestaltung wird der Hemmstoff direkt z.B. über die Versorgungsleitung zu der jeweiligen Zelllinie gegeben, d.h. nur die Zellen in diesem Modul kommen mit dem Hemmstoff in Kontakt. Sind hinter dieses Modul weitere Module geschaltet, besteht die Möglichkeit, dass nachfolgende Zellen mit dem Überschuss des Hemmstoffs in der Lösung des Fluidikkreislaufs in Kontakt kommen, wenn dieser die Membran passieren kann. In einer bevorzugten Ausgestaltung ist die Membran für den Hemmstoff nicht permeabel, d.h. er kann sie nicht passieren und verbleibt in der Zellkammer des Moduls zu dem er hinzugefügt wurde.

Werden Temperatur oder Lichtsignale oder elektrische Signale als Aktivatoren oder Hemmstoffe eingesetzt, gelangen diese von außen in das System und das Material des mikrofluidischen Bioreaktors muss entsprechend ausgewählt werden.

Die Zellkammern dienen zur Aufnahme von natürlichen oder transgenen Zellen pflanzlicher oder tierischer Zelllinien, Protoplasten, Hefen, Pilze oder Bakterien, bevorzugt natürlicher oder transgener Pflanzenzellen oder Protoplasten. In einer Ausgestaltung lässt sich die Zellkammer öffnen, so dass die Zellen von außen, bevorzugt als Suspension, in die Zellkammer eingebracht werden. In einer weiteren Ausgestaltung verfügt jede Zellkammer über eine Versorgungs- und eine Abflussleitung, die es ermöglicht Zellen, suspendiert in einer Flüssigkeit, bevorzugt einem Zellkulturmedium, in das System einzubringen. Die Abflussleitung ermöglicht dabei einen Abfluss des überflüssigen Flüssigkeitsvolumens. In einer weiteren Ausgestaltung des mikrofluidischen Bioreaktors sind mehrere Zellkammern über eine gemeinsame Versorgungsleitung mit einem gemeinsamen Versorgungsgefäß verbunden und verfügen über eine gemeinsame Abflussleitung. Die Abflussleitung des vorhergehenden Moduls stellt dabei die Versorgungsleitung des nachfolgenden Moduls dar. Diese Ausgestaltung ermöglicht das Befüllen mehrerer Zellkammern mit Zellen der gleichen Zelllinie. Die Zellen, suspendiert in einer Flüssigkeit werden so über ein Versorgungsgefäß in die Zellkammer eines Moduls und weiter in die Zellkammer der nachfolgenden Module gespült. In einer weiteren Ausgestaltung verfügt die Zellkammer nicht über eine-Abflussleitung, überschüssige Flüssigkeit wird dabei über die Verbindungsleitung der Stoffkammer abtransportiert.

Pflanzliche Zellen benötigen keine Adhäsion an einem Untergrund für ihr Wachstum. Die Zellen sind zunächst frei schwebend in der Zellkammer und setzen sich dann aufgrund ihres spezifischen Gewichts ab. In einer bevorzugten Ausgestaltung ist das Modul so aufgebaut, dass die Zellen sich durch die Schwerkraft von oben auf der Membran absetzen. Die Zellkammer wird nach dem Einbringen der Zellen verschlossen. Für die Verwendung von Zelllinien, die eine Adhäsion an die Zellkammer, bzw. die Membran benötigen, werden die Adhäsion unterstützende Materialien und Beschichtungen eingesetzt. Die Auswahl erfolgt abhängig von den verwendeten Zelllinien aus den bekannten Materialien und Beschichtungen.

Da durch die modulare Konstruktion des Bioreaktors alle Zellkammern separat befüll-, separat entleer- und mit einem konstanten Fluss durchströmbar sind und die Zellen sich in den entsprechenden Zellkammern absetzten, sind keine speziellen Rückhalte- oder Entladungsvorrichtungen notwendig. So ist es auch möglich, die mit Zellen beladene Kammer konstant, bevorzugt mit kleinem Volumenstrom zu durchspülen, ohne dass sich die Zellen wegbewegen.

In einer weiteren Ausgestaltung ist mindestens eine der beiden Leitungen, der Versorgungs- und der Abflussleitung, so mit einer druckerzeugenden Quelle verbunden, dass die Zellen, suspendiert in einer Flüssigkeit in die Zellkammer gepumpt und/oder die überschüssige Flüssigkeit aus der Zell- oder der Stoffkammer gepumpt wird. In einer besonders bevorzugten Ausgestaltung kann der Druck auf die jeweilige Zelllinie angepasst werden, um Zerstörung der Zellen zu vermeiden.

Es werden flache Geometrien der Zellkammer bevorzugt, da die am weitesten von der Membran entfernt liegenden Zellen nur per Diffusion mit dem Reaktanden aus der Stoffkammer versorgt werden. Die Höhe der Zellkammer, der Abstand zwischen der Membran und der ihr gegenüberliegenden, begrenzenden Wand der Zellkammer, entspricht der Höhe mehrerer Lagen der zu verwendeten Zelllinien. Bevorzugt soll die Höhe der Zellkammer maximal 20-mal der längsten Ausdehnung der zu verwendenden Zelllinie betragen, besonders bevorzugt maximal 10-mal der längsten Ausdehnung der zu verwendenden Zelllinie betragen. Der Einsatz von kleinen Kammer- und Kanalsystemen in mikrofluidischer Fertigungsweise ist aufgrund der limitierenden Diffusionswege zwischen den Zellen erforderlich. In einer Ausgestaltung ist die Zellkammer daher 0,01 mm - 5 mm, bevorzugt 0,05 mm - 2,5 mm und besonders bevorzugt 0,1 - 1 mm hoch. Die Stoffkammer weist dieselbe Höhe oder eine von der Zellkammer abweichende Höhe auf. Die Zellkammer ist in einer Ausgestaltung 0,01 mm - 5 mm, bevorzugt 0,05 mm - 2,5 mm und besonders bevorzugt 0,1 - 1 mm hoch. Die maximale Ausdehnung der Zellkammer und der Stoffkammer, d.h. die Fläche über die die Zellkammer und die Stoffkammer mit der Membran verbunden sind, beträgt 10 mm² - 5000 mm², bevorzugt 50 mm² - 2500 mm² und besonders bevorzugt 100 mm² - 1000 mm². Das befüllbare Volumen der Zellkammer beträgt 10 µl - 5000 µl, bevorzugt 20 µl - 2000 µl und besonders bevorzugt 50 µl - 1000 µl.

In einer bevorzugten Ausgestaltung des mikrofluidischen Bioreaktors werden die einzelnen Module durch ein Stecksystem miteinander in Kontakt gebracht. Dabei sind die einzelnen Module voneinander trennbar, d.h. die Zuleitungen und Ableitungen der Stoffkammern der einzelnen Module sind mit Anschlüssen versehen, die sich über Verbindungsstücke miteinander so in Kontakt bringen lassen, dass die flüssige Lösung mit dem mindestens einem Zusatzstoffen im Fluidleitungssystem vom Vorratsgefäß über die Stoffkammer des ersten Moduls in die Stoffkammer des zweiten Moduls in die Stoffkammern der eventuell weiteren vorhandenen Module befördert wird. Der Vorteil eines Stecksystem besteht darin, dass bereits mit Zellen beladene Module je nach Reihenfolge der Syntheseschritte zusammengesteckt werden können und nach Synthese eines Zellmetaboliten die Reihenfolge der hintereinander geschalteten Zelllinien in den einzelnen Zellkammern der Module beliebig geändert werden kann, ohne die Zellkammern neu beladen zu müssen. Bevorzugt erfolgt der Zusammenbau mittels Stecksystem so, dass die Versorgungsleitungen in eine jeweils passende Aufnahme der Kammereinheit geschoben werden, wobei eine Abdichtung über O-Ringe erfolgt.

In einer weiteren Ausgestaltung werden mehrere Module des mikrofluidischen Bioreaktors auf einen gemeinsamen Träger aufgebracht, so dass sie nicht über ein Stecksystem variabel mit einander in Verbindung gebracht werden, sondern permanent dieselbe Reihenfolge aufweisen. Diese Ausgestaltung eignet sich vor allem für Standardsynthesen mit einem immer gleichen Aufbau.

In einer besonders bevorzugten Ausgestaltung weist das Fluidleitungssystem der Stoffkammern einzeln absperrbare Ventile auf. So besteht die Möglichkeit, einzelnen Module im laufenden Betrieb, d.h. wenn der mikrofluidische Bioreaktor flüssigkeitsgefüllt ist, aus dem System zu entfernen oder die Reihenfolge der einzelnen Module zu ändern.

Alle Herstellungsprozesse, Arbeitsmittel und Materialien in Bezug auf den Bioreaktor müssen möglichst biokompatibel und sauber sein. Die mikrofluidischen Strukturen werden aus Kunststoffen, d.h. Polymeren, Gläsern oder Metallen bevorzugt aus Polymeren hergestellt, so dass zu deren Herstellung geeignete Verfahren, wie z.B. Abformung (Heißprägen, Spritzgießen), direktes Fräsen, 3D-Druck, Gießen, Spritzgießen, Ätztechnik, Lithographie oder Rapid-Prototyping eingesetzt werden.

Der mikrofluidische Bioreaktor wird bevorzugt aus thermoplastischen, biokompatiblen Polymeren erzeugt. Besonders bevorzugt werden die beiden Einheiten aus Polycarbonat (PC), Polymethylmethacrylat (PMMA), Cycloolefincopolymer (COC) oder Polydimethylsiloxan (PDMS), Polystyrol (PS), Polysulfon (PSÜ), Polyethylenterephtalat (PET), Polytetrafluorethylen(PTFE), Polypropylen (PP) oder Polyethylen (PE) oder Mischungen daraus hergestellt.

Die Verbindung der beiden Einheiten mit der Membran wird über Standardmethoden, ausgewählt unter thermisches Bonden, Verkleben, Zusammenpressen oder Ultraschallschweißen realisiert. Dabei werden Polymerwerkstoffe mit Membranen aus demselben Polymer bevorzugt thermisch gebondet, ultraschallverschweißt oder geklebt. Bei einer Ausgestaltung in der die beiden Einheiten aus einem anderen Polymer hergestellt wurde als die verwendete Membran, oder bei Verwendung einer Metallmembran, erfolgt die Verbindung über Ultraschallschweißen oder Kleben. Glas- bzw. Metall-Einheiten werden mit der Membran verklebt. In einer Ausgestaltung werden beide Einheiten aus dem gleichen Material hergestellt. In einer bevorzugten Ausgestaltung ist das Material der Membran das gleiche Material, wie das der beiden Einheiten.

In einer Ausgestaltung werden die beiden Einheiten mittels Rapid-Prototyping-Verfahren aus Epoxidharzen hergestellt, die dann mittels Kleben mit der Membran in Verbindung gebracht werden.

Einschränkungen ergeben sich beim thermischen Bonden, da hier nur Materialien verwendet werden können, die auch als Membran zur Verfügung stehen, da Gehäuse und Membran aus dem gleichen Material bestehen sollten.

In einer weiteren Ausgestaltung werden die beiden Einheiten in PDMS abgeformt und die Teile dann zusammen mit einer porösen Membran zusammengedrückt. PDMS ist elastisch und dichtet durch das Zusammendrücken mit dafür geeigneten Klammervorrichtungen ab.

In einer weiteren Ausgestaltung werden die Einheiten aus Foturan®, einer Glassorte, die mittels optischer Lithografie strukturiert und anschließend geätzt werden kann, hergestellt. Das Ätzen von Glas ist auch mit einer strukturierten Abdeckschicht möglich. In beiden Fällen erhält man optisch intransparente Oberflächen.

In einer Ausgestaltung wird der mikrofluidische Bioreaktor zumindest teilweise, bevorzugt zumindest im Reaktionsbereich, aus einem transparenten Material erzeugt, um entweder die Zellkultur mikroskopisch zu beobachten oder um Lichtsignale einzukoppeln. In einer weiteren Ausgestaltung werden nicht transparente oder eingefärbte Kunststoffe verwendet, wenn das Zellwachstum unter bestimmten Beleuchtungsbedingungen erfolgen soll.

Um flexibel die Reaktionsbedingungen steuern zu können, werden in besonders bevorzugten Ausgestaltungen des mikrofluidischen Bioreaktors einzelne Module mit kälte- oder wärmeerzeugenden Einrichtungen versehen. Dazu finden bevorzugt Kälte- oder Heizspiralen oder Peltierelemente Einsatz.

Die verwendete Membran ist durchlässig, d.h. sie ermöglicht den stofflichen Austausch zwischen der Zellkammer und der Stoffkammer. Dazu weist die Membran in einer Ausgestaltung Poren auf. Durch die Wahl einer geeigneten Porengröße kann die Größe der wandernden Moleküle und Zellen beschränkt werden, d.h. Moleküle oder auch Zellen, die größer als die gewählte Porengröße sind, können nicht durch die Membran gelangen. Die Wahl der Porengröße wird auch durch die verwendete Zelllinie bestimmt. Die Zellgrößen sind je nach verwendeter Zellkultur sehr unterschiedlich. Die Zellen der BY-2 Tabakzelllinie (*Nicotiana tabacum* L. cv Bright Yellow 2; [9]) haben eine durchschnittliche Länge von 55 µm und eine durchschnittliche Breite von 35 %m. Bei anderen Zelllinien gibt es auch wesentlich größere Zellen, wie bei der Tabakzelllinie VBI-0 (*Nicotiana tabacum* L. cv Virginia Bright Italia; [10]), die bis zu 150 µm lang und 75 µm breit werden. Daneben werden auch Zellkulturen mit wesentlich kleineren Zellen verwendet, wie z.B. *Arabidopsis thaliana* (L. var. Landsberg, [11]) oder Reiszellsuspensionskulturen [12]. Die gewählte Porengröße verhindert einen Übergang der Zellen der verwendeten Zelllinie von der Zellkammer in die Stoffkammer, indem die Poren der Membran im Durchmesser kleiner sind, als die verwendete Zelllinie. Die Porendichte, d.h. die Anzahl an Poren pro Fläche der Folie hängt ebenfalls von der zu verwendenden Zelllinie ab. Bei kleinen Poren ist eine hohe Porendichte bevorzugt. Folien mit großen Poren sollten eine geringere Porendichte aufweisen, um einerseits Risse, und damit vergrößerte Poren, beim Anlegen des Drucks mit dem die Flüssigkeit durch den zweiten Flüssigkeitskreislauf bewegt wird, zu vermeiden, aber andererseits auch um vergrößerte Porendurchmesser bei zu eng benachbarten Poren zu vermeiden.

Die verwendete Membran ist bevorzugt aus Polymeren hergestellt. In einer besonders bevorzugten Ausgestaltung werden ionengespurte Membranen eingesetzt.

In einer weiteren, bevorzugten Ausgestaltung werden Membranen aus Polymeren eingesetzt, die semi-permeabel sind, d.h. die bestimmte Stoffe nur in bestimmte Richtungen durchlassen. Dadurch wird ermöglicht, dass nur ausgewählte Zusatzstoffe aus dem zweiten Fluidikkreislauf in die Zellkammer gelangen, und dass nur ausgewählte Zusatzstoffe aus der Zellkammer in den zweiten Fluidikkreislauf gelangen.

In einer weiteren Ausgestaltung ist die Membran aus Metall hergestellt und weist eine Mikrosiebstruktur auf.

Die verschiedenen Ausgestaltungen können frei miteinander kombiniert werden.

Um Zellmetaboliten mit dem mikrofluidischen Bioreaktor zu gewinnen, werden die Zellen nach Verfahrensschritt a) in die Zellkammern eingebracht. Dies erfolgt in einer Ausgestaltung indem die Zellkammer geöffnet wird und die Zellen von außen in die Zellkammer eingebracht werden. Die Zellkammer wird anschließend flüssigkeitsdicht verschlossen. In einer weiteren Ausgestaltung werden die Zellen über die Versorgungsleitung in das System eingebracht, indem sie unter Anlegen von Druck aus einem Vorratsgefäß über die Versorgungsleitung in die Zellkammer geschleust werden. Anschließend wird nach Verfahrensschritt b) ein Flüssigkeitsstrom einer flüssigen Lösung mit mindestens einem Zusatzstoff im Fluidleitungssystem des mikrofluidischen Bioreaktors zur Synthese des mindestens einen Zellmetaboliten angelegt. Dadurch dringt die flüssige Lösung mit dem mindestens einem Zusatzstoff durch die Membran aus der Stoffkammer in die Zellkammer ein. In der Zellkammer wird mit den Zellen mindestens ein Zellmetabolit synthetisiert und die flüssige Lösung mit dem mindestens einem Zusatzstoff und dem mindestens einem Zellmetabolit gelangt durch die Membran zurück in das Fluidleitungssystem.

Die flüssige Lösung im Fluidikkreislauf enthält mindestens einen Zusatzstoff. Die Syntheseschritte in den einzelnen Kammern werden in bevorzugten Ausgestaltungen unter Einwirkung von Hemmstoffen und Aktivatoren beeinflusst. Dazu werden diese Zusatzstoffe entweder zur flüssigen Lösung im Fluidikkreislauf hinzugefügt oder in die einzelnen Module direkt appliziert. Ebenso erfolgt die Versorgung der Zelllinien entweder über Nährstoffe, die zur flüssigen Lösung im Fluidikkreislauf hinzugefügt werden oder indem die Nährstoffe direkt in die einzelnen Module appliziert werden. Die Zugabe von Zusatzstoffen direkt in die einzelnen Module erfolgt über die geöffnete Zellkammer, die Versorgungsleitung oder zusätzliche Leitungen, die direkt in die Zellkammer führen.

Die Synthese im ersten Modul startet, sobald der Reaktand in der flüssigen Lösung des Fluidikkreislaufs aus der Stoffkammer des ersten Moduls in die Zellkammer des ersten Moduls wandert und dort von den Zellen in ein Produkt umgewandelt wird. Dieses Produkt wird dann von den Zellen in die flüssige Lösung des Fluidikkreislaufs abgegeben und über das Fluidleitungssystem vom ersten in das nächste Modul geschleust. Die das zweite Modul erreichende Lösung enthält nun unumgesetzte Reste des ursprünglichen Edukts, das Produkt der Synthese im ersten Modul und eventuell weitere Zusatzstoffe. Das Produkt der Synthese des ersten Moduls gelangt nun über die Membran des zweiten Moduls in die Zellkammer des zweiten Moduls und wird dort von den Zellen aufgenommen und in ein weiteres Produkt, das Produkt der Synthese im zweiten Modul, umgewandelt. Dieser Prozess wird so oft wiederholt, wie es hintereinandergeschaltete Module im mikrofluidischen Bioreaktor gibt. Das Produkt der Synthese des letzten Moduls stellt das Gesamtprodukt der Synthese, den Zellmetabolit dar. Der Zellmetabolit kann dann entsprechend dem Verfahrensschritt c) aus dem Flüssigkeitsstrom entnommen werden. Dazu schließt sich in einer Ausgestaltung an das letzte Modul ein Sammelgefäß an. In einer weiteren Ausgestaltung wird die flüssige Lösung des zweiten Fluidikkreislaufs mit dem Zellmetaboliten direkt zu mindestens einer Reinigungseinrichtung, ausgewählt unter präparativer oder semipräparativer Chromatographie, Elektrophorese, Extraktion, Fällung, Filtration, Sedimentation oder Evaporation zugeführt. In einer bevorzugten Ausgestaltung erfolgt eine Aufreinigung unmittelbar aus der flüssigen Lösung. Dafür werden die Anlagen, welche die Reinigungsschritte durchführen direkt über die Ablaufleitung des erfindungsgemäßen mikrofluidischen Bioreaktors versorgt. In einer besonders vorteilhaften Ausgestaltung wird der mikrofluidische Bioreaktor direkt in ein Lab-on-the-Chip integriert. Die in den einzelnen Modulen verwendeten Zelllinien sind gleiche oder verschiedene Zelllinien, die gleiche oder verschiedene Syntheseschritte durchführen. Das Produkt jedes einzelnen Syntheseschritts hängt von der verwendeten Zelllinie, dem Reaktand, den Reaktionsbedingungen und den Aktivatoren und Hemmstoffen sowie den weiteren Zusatzstoffen, die in diesem Modul in Kontakt mit den Zellen kommen, ab. Die Auswahl erfolgt jeweils hinsichtlich des durchzuführenden Syntheseschritts.

Um lichtsensitive Reaktionen durchführen zu können, werden einzelne Module aus gefärbten, lichtabweisenden Materialien verwendet. Bei photochemischen Reaktionen werden Module aus transparenten, die jeweilig benötigte Wellenlänge durchlassende Materialien verwendet. Innerhalb der Reaktionskette werden je nach Anforderungen der Syntheseschritte einzelne Module über wärme- oder kälteerzeugende Einrichtungen erwärmt oder abgekühlt.

Mit dem mikrofluidischen Bioreaktor lassen sich Zellmetaboliten durch Kombination verschiedenster Zelllinien herstellen. Es finden pflanzlichen oder tierische Zelllinien Einsatz, die sowohl natürlichen als auch genetisch veränderten Ursprungs sind.

Für die Herstellung von Zellmetaboliten mit dem Bioreaktor werden Zellen im Wesentlichen auf drei Weisen genetisch verändert: 1. Regulation der Gene, die für Schlüsselenzyme der entsprechenden Stoffwechselwege kodieren. 2. Beeinflussung des Sekundärstoffwechsels durch Einbringen neuer Gene. 3. Veränderung des Sekundärstoffwechsels durch Herunterregulation oder Überexpression spezifischer Stoffwechselgene. [13]

Die Erfindung wird im Folgenden anhand der folgenden Figuren und Ausführungsbeispielen näher erläutert.
- Fig. 1: Schematischer Aufbau des mikrofluidischen Bioreaktors
- Fig. 2: Explosionszeichnung eines Moduls des Bioreaktors
- Fig. 3: Schematischer Ablauf des erfindungsgemäßen Verfahrens zur Gewinnung von Zellmetaboliten
- Fig. 4: Ausgestaltungsvarianten der ersten Einheit eines Moduls
- Fig. 5: Ausgestaltungsvarianten mit paralleler und kombinierter paralleler-linearer Kopplung der einzelnen Module
- Fig. 6: Zellvitabilität der Zellen aus Ausführungsbeispiel 4
- Fig. 7: Bestimmung des Mitoseindex aus Ausführungsbeispiel 4
- Fig. 8: Zell-Zell-Kommunikation aus Ausführungsbeispiel 4

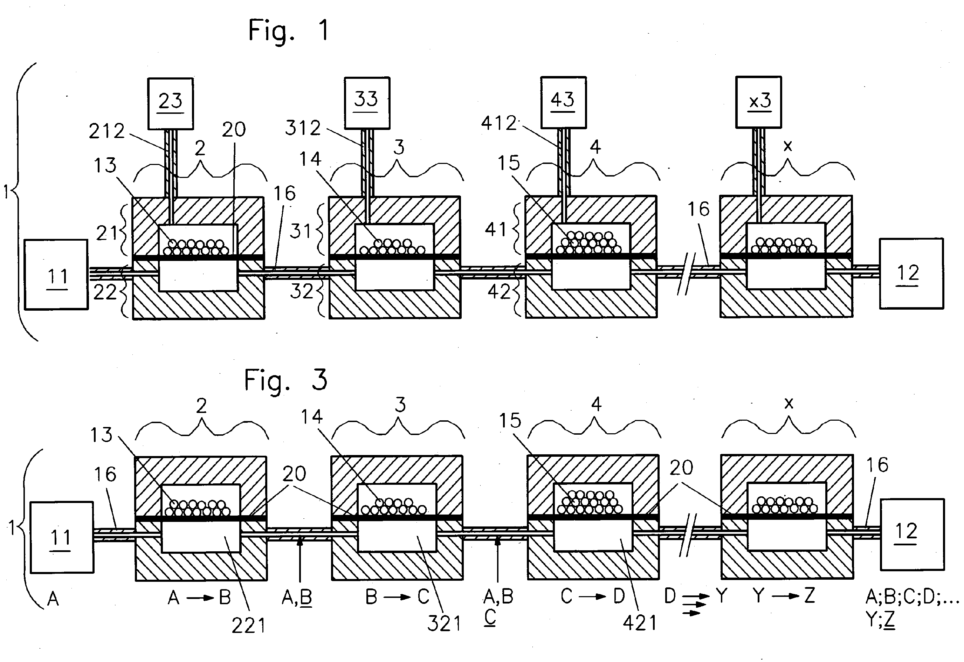

**Fig.** 1 zeigt den prinzipiellen Aufbau des mikrofluidischen Bioreaktors (1) mit mehreren Modulen (2, 3, 4). Das erste Modul (2) bestehen aus jeweils 2 Einheiten (21, 22). Die erste Einheit (21) stellt die Zellkammer, welche über die Versorgungsleitungen (212) aus den Versorgungsgefäßen (23) mit den Zellen (13) befüllt werden. Das zweite Modul (3) bestehen aus jeweils 2 Einheiten (31, 32). Die erste Einheit (31) des zweiten Moduls (3) stellt die Zellkammer, welche über die Versorgungsleitungen (312) aus den Versorgungsgefäßen (33) mit den Zellen (14) befüllt werden. Die weiteren Module (4, X) bestehen ebenfalls aus jeweils 2 Einheiten (41, 42). Die Synthese des Zellmetaboliten startet, sobald aus dem Vorratsgefäß (11) die flüssige Lösung mit dem mindestens einem Zusatzstoff über das Fluidleitungssystem (16) in die Stoffkammer der zweiten Einheit (22) des ersten Moduls (2) und von dort in die weiteren zweiten Einheiten (32, 42) der weiteren Module (3, 4, X) gelangt und der Reaktand zur Synthese des Zellmetaboliten über die Membranen (20) in die Zellkammern wandert und dort von den Zellen metabolisiert wird. Der gewünschte Zellmetabolit kann dann aus dem Sammelgefäß (12) entnommen werden

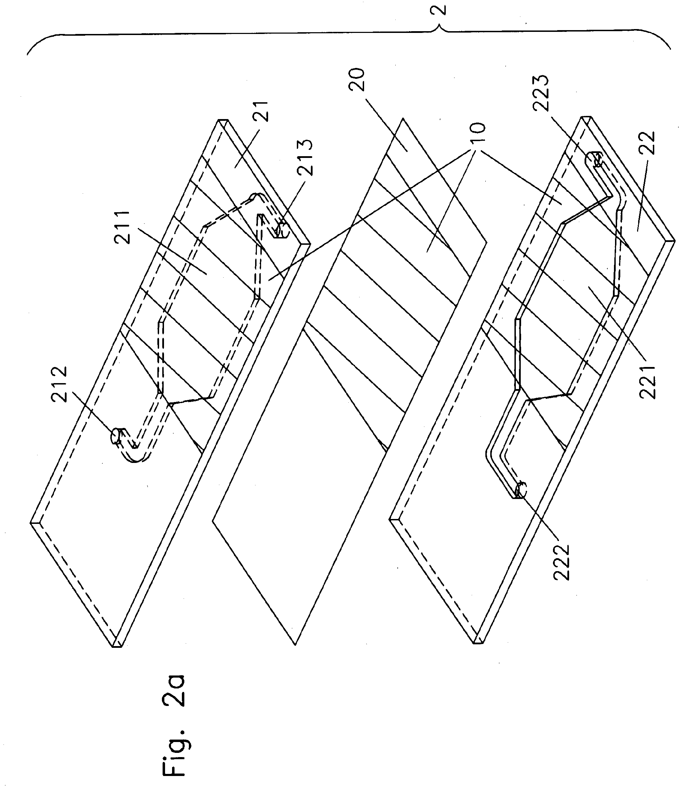

**Fig. 2a** zeigt eine Explosionszeichnung eines Modelles eines Moduls (2) des mikrofluidischen Bioreaktors, mit einer ersten Einheit (21) mit einer Zellkammer (211) und einer zweite Einheit (22) mit einer Stoffkammer (221), die formschlüssig zur Zellkammer (211) der ersten Einheit (21, 31, 41) angeordnet ist und einer Membran (20), die so zwischen die erste Einheit (21), und die zweite Einheit (22) eingebracht ist, dass sie die Zellkammer (211) von der Stoffkammer (221) trennt und die im Reaktionsbereich (10) zum in Kontakt bringen des Reaktanden in der flüssigen Lösung der Stoffkammern (221) mit den Zellen in der Zellkammer (21) zumindest teilweise durchlässig ist. Des Weiteren verfügt die Stoffkammer über eine Zuflussleitung (222) und eine Verbindungsleitung (223) über die sie in das Fluidleitungssystem des mikrofluidischen Bioreaktors eingebunden ist.

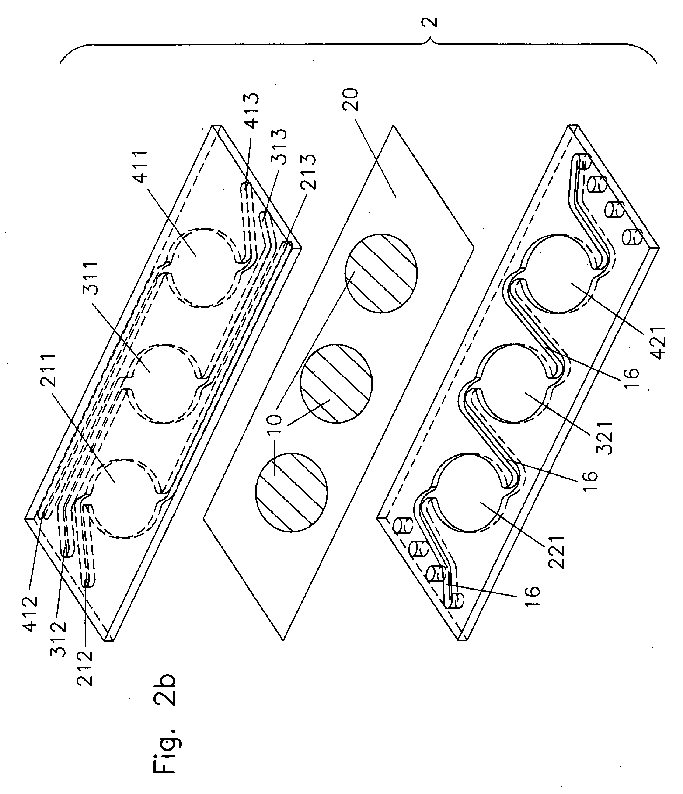

**Fig. 2b** zeigt eine Explosionszeichnung eines Modelles eines Kombination von Modulen des mikrofluidischen Bioreaktors (1), mit drei Zellkammern (211, 311, 411) und drei Stoffkammern (221, 321, 421) und einer Membran (20), die so zwischen den drei Zellkammern (211, 311, 411) und den drei Stoffkammern (221, 321, 421) eingebracht ist, dass sie die Zellkammern (211, 311, 411) von den Stoffkammern (221, 321, 421) trennt und die im Reaktionsbereich (10) zum in Kontakt bringen des Reaktanden in der flüssigen Lösung der Stoffkammern (221, 321, 421) mit den Zellen in der Zellkammer (211, 311, 411) zumindest teilweise durchlässig ist. Die erste Zellkammer (211) liegt dabei formschlüssig auf der ersten Stoffkammer (321). Die zweite Zellkammer (311) liegt dabei formschlüssig auf der zweiten Stoffkammer (321). Die dritte Zellkammer (411) liegt dabei formschlüssig auf der dritten Stoffkammer (421). Des Weiteren sind die Stoffkammern in das Fluidleitungssystem (16) des mikrofluidischen Bioreaktors eingebunden. Des Weiteren verfügen die Zellkammern (211, 311, 411) über jeweils eine Versorgungs- (212, 312, 412) und eine Abflussleitung (213, 313, 413).

**Fig. 3** zeigt das Grundprinzip der Synthese eines Zellmetaboliten mit dem mikrofluidischen Bioreaktor. Die Stoffkammer (221) des ersten Moduls (2) wird mit der flüssigen Lösung des Fluidikkreislauf des Fluidleitungssystems (16) aus dem Vorratsgefäß (11) durchströmt. Die flüssige Lösung aus dem Vorratsgefäß enthält mindestens einen Zusatzstoff, den Reaktand A. Beim Durchströmen des Moduls (2) wird der Reaktand A von der Stoffkammer (221) in die Zellkammer (211) befördert und kommt dort mit den Zellen (13) in Kontakt. Die Zellen (13) setzen A zu B um und geben B in den Fluidikkreislauf des Fluidleitungssystems (16) ab. Die flüssige Lösung, die aus dem Modul (2) herausströmt, enthält sowohl den überschüssigen Reaktanden A als auch das neu hergestellte Produkt B. Beim Durchströmen des nächsten Moduls (3) wird das Produkt B (jetzt Reaktand B) von der Stoffkammer (321) in die Zellkammer (311) befördert und kommt dort mit den Zellen (14) in Kontakt. Die Zellen (14) setzen B zu C um und geben C in den Fluidikkreislauf des Fluidleitungssystems (16) ab. Die flüssige Lösung, die aus dem Modul (3) herausströmt, enthält sowohl die überschüssigen Reaktanden A und B als auch das neu hergestellte Produkt C. Beim Durchströmen des nächsten Moduls (4) wird das Produkt C (jetzt Reaktand C) von der Stoffkammer (421) in die Zellkammer (411) befördert und kommt dort mit den Zellen (15) in Kontakt. Die Zellen (15) setzen C zu D um und geben D in den Fluidikkreislauf des Fluidleitungssystems (16) ab. Die flüssige Lösung, die aus dem Modul (4) herausströmt, enthält sowohl die überschüssigen Reaktanden A, B und C als auch das neu hergestellte Produkt D. Je nach Anzahl der Module wiederholt sich das Ganze, bis das Produkt Y, der Reaktand für die Synthese von gewünschten Zellmetaboliten hergestellt und an die flüssige Lösung abgegeben wurde. Beim Durchströmen des letzten Moduls (X) wird das Produkt Y (jetzt Reaktand Y) von der Stoffkammer in die Zellkammer befördert und kommt dort mit den Zellen in Kontakt. Die Zellen setzen Y zum gewünschten Zellmetaboliten Z um und geben diesen in den Fluidikkreislauf des Fluidleitungssystems (16) ab. Die flüssige Lösung, die aus dem Modul (X) herausströmt, enthält sowohl die überschüssigen Reaktanden A, B, C, bis Y als auch das neu hergestellte Produkt, den gewünschten Zellmetaboliten Z. Die flüssige Lösung wird dann über den Fluidikkreislauf des Fluidleitungssystems zu einer Aufreinigungsgsanlage oder einem Sammelgefäß (12) weitertransportiert.

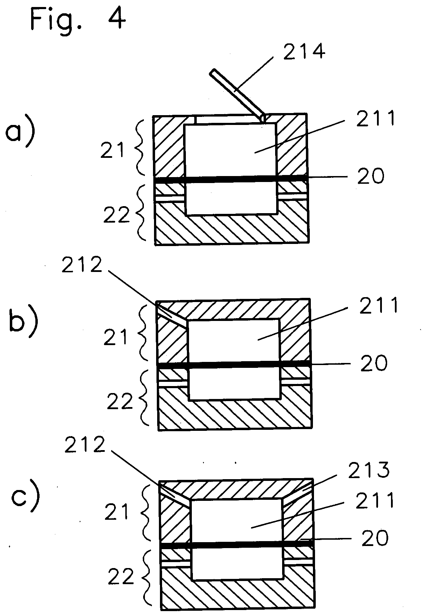

**Fig. 4** zeigt verschiedene schematisch die Ausgestaltungen der ersten Einheit (21) eines Moduls (2) des mikrofluidischen Bioreaktors (mit der zweiten Einheit (22) und der Membran (20))mit a) einer Befüllung der Zellkammer (211) durch Öffnen der Zellkammer mittels eines Deckels (214); b) einer Befüllung durch eine eigene Versorgungsleitung (212) , wobei der Abfluss überschüssiger Flüssigkeit über die Stoffkammer erfolgt; c) einer Befüllung durch eine eigene Versorgungsleitung (212). Der Abfluss erfolgt über eine eigene Abflussleitung (213).

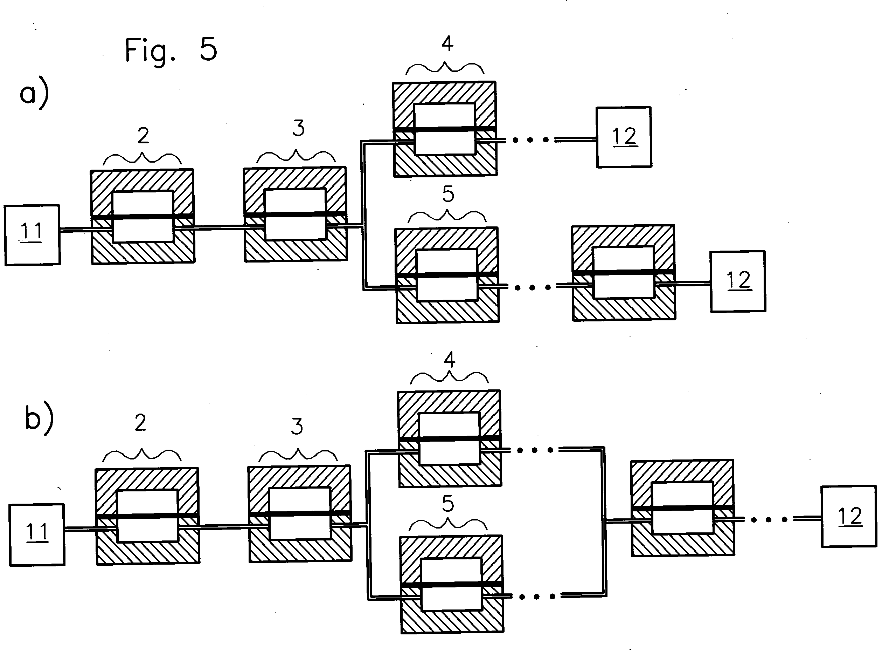

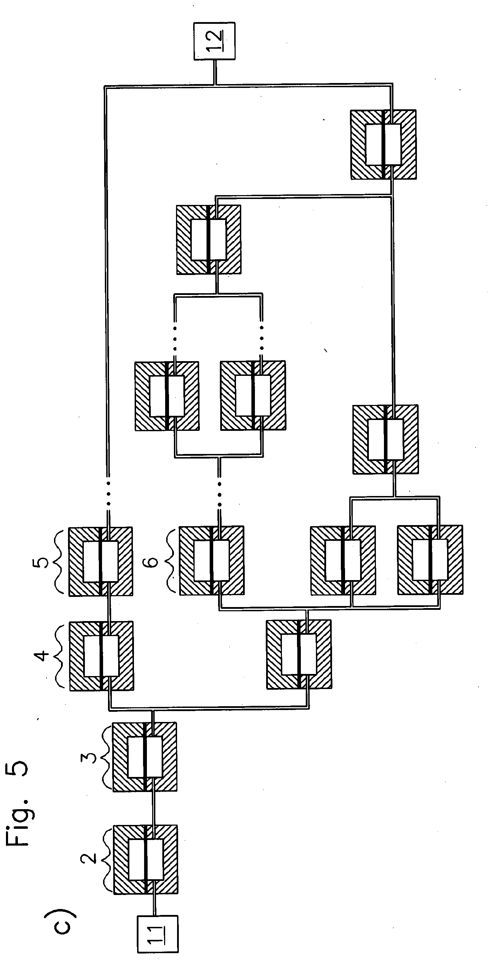

**Fig. 5** zeigt Ausgestaltungen mit paralleler und kombinierter paralleler-linearer Kopplung der einzelnen Module (2, 3, 4, 5, 6) des mikrofluidischen Bioreaktors angeschlossen an ein Vorratsgefäß (11) und ein Sammelgefäß (12): a) zeigt eine parallele Kopplung zur Synthese mehrerer verschiedener Zellmetaboliten, b) zeigt eine lineare-parallele Kopplung mit einem Verzweigungspunkt zur Synthese eines Zellmetaboliten, wobei während dessen Synthese 2 verschiedene Reaktanden aus einem Edukt hergestellt werden , c) zeigt eine lineare-parallele Kopplung mit mehreren Verzweigungspunkten.

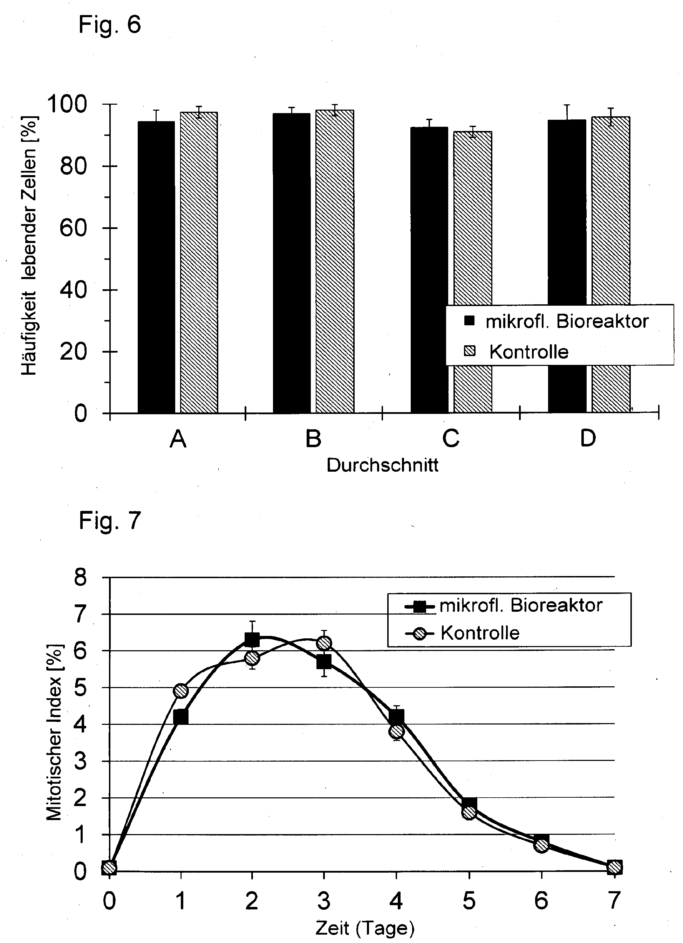

**Fig. 6** zeigt die Zellvitabilität der Zellen aus Ausführungsbeispiel 4.

**Fig. 7** zeigt die Bestimmung des Mitoseindex aus Ausführungsbeispiel 4. In der exponentiellen Wachstumsphase (Tag 1 bis 4) lag der Mitoseindex bei beiden Ansätzen zwischen 4 und 6,5 %.

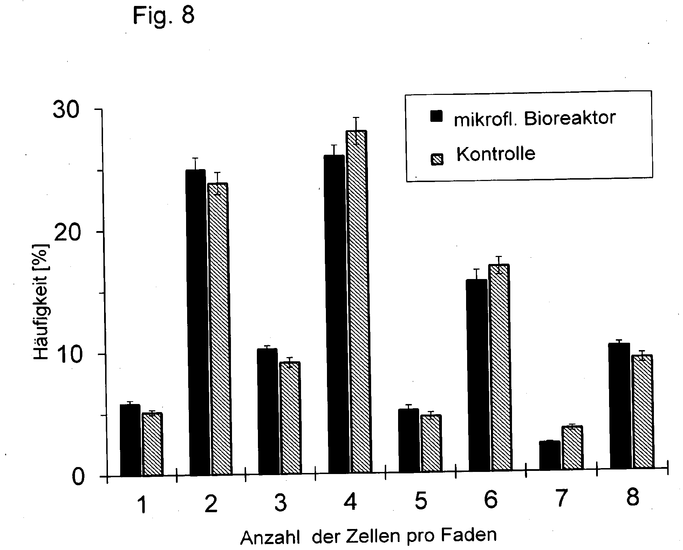

**Fig. 8** zeigt die Zell-Zell-Kommunikation und das koordinierte Wachstum aus Ausführungsbeispiel 4. Die charakteristischen Maxima hinsichtlich der Häufigkeitsverteilung von zweizelligen (25 %), vierzelligen (27 %) und sechszelligen Fäden (16 %) der 4 Tage alten Kultur waren bei beiden Versuchsansätzen nachweisbar.

### Beispiel 1: mikrofluidischer Bioreaktor mit sechs-eckiger Kammergeometrie

Mikrofluidischer Bioreaktor hergestellt aus Polycarbonat (PC) mit einer rechteckigen Grundfläche von 26 mm x 76 mm und 2 mm Stärke. Die Höhe pro Einheit beträgt 1 mm. Die Zell-/Stoffkammer sind identisch zueinander, weisen eine 6-eckige Form auf und bieten mit den Abmaßen 15 mm (Breite) x 27,5 mm (Länge) x 0,5 mm (Höhe) eine Fläche von 300 mm². Das befüllbare Volumen der Zellkammer beträgt 150 µl. Die Kammern wurden durch Heißprägen hergestellt.

Die verwendete Membran, eine PC-Filtermembran mit 0,4 µm Poren ist "teilporös" d.h. sie ist nur im Bereich der Zell-/Stoffkammer porös. Der mikrofluidische Bioreaktor wurde mit einem Fluss von 75 µl/min betrieben.

### Beispiel 2: mikrofluidischer Bioreaktor mit drei elliptischen Kammern auf einem Träger ohne Steckverbindung

Mikrofluidischer Bioreaktor hergestellt aus Polycarbonat (PC) mit einer rechteckigen Grundfläche von 26 mm x 76 mm und 2 mm Stärke. Die Zell-/Stoffkammer sind identisch zueinander, weisen eine elliptische Form auf und bieten mit einem Ellipsenradius zwischen 6 mm und 9 mm und einer Höhe von 0,5 mm eine Fläche von 170 mm². Die Kanäle sind 1,5 mm breit und 0,5 mm hoch Das befüllbare Volumen der Zellkammer beträgt 85 µl. Die Strukturen wurden durch direktes Fräsen in das PC-Grundmaterial erhalten. Die verwendete Membran ist eine poröse PC-Filtermembran mit 0,4 µm Poren. Die beiden Einheiten wurden mittels Ultraschallschweißen mit der Membran verbunden. Der mikrofluidische Bioreaktor wurde mit einem Fluss von 75 µl/min betrieben.

### Beispiel 3: mikrofluidischer Bioreaktor mit einer elliptischen Kammer

Mikrofluidischer Bioreaktor hergestellt aus Polycarbonat (PC) mit einer elliptischen Grundfläche von 10,5 x 26,8 mm und 2 mm Stärke. Die Zell- und die Stoffkammer sind identisch zueinander, weisen eine elliptische Form auf und bieten mit Ellipsenradien von 7,5 mm bis 23,8 mm eine Fläche von ca. 561 mm². Die Zellkammer ist 0,5 mm hoch, die Stoffkammer 1 mm. Die Kanäle sind 1,5 mm breit und 0,5 mm hoch. Das befüllbare Volumen der Zellkammer beträgt 280 µl. Die verwendete Membran ist eine PC-Filtermembran mit 0,4 µm Poren. Die Strukturen wurden durch direktes Fräsen in das PC-Grundmaterial erhalten. Die beiden Einheiten wurden mittels Ultraschallschweißen mit der Membran verbunden. Der mikrofluidische Bioreaktor wurde mit einem Fluss von 75 µl/min betrieben.

### Beispiel 4 Zellzucht von Tabak-BY2-Zellen:

Es wurde der mikrofluidischer Bioreaktor aus Beispiel 1 mit 70% Ethanol sterilisiert und dann mit sterilem destilliertem Wasser gespült. Anschließend wurde der mikrofluidische Bioreaktor mit sterilem MS-Medium (Zusammensetzung des Mediums: [10]) gefüllt. Die Tabak-BY2 Zellen (*Nicotiana tabacum* L. cv Bright Yellow 2) wurden jeweils einer Suspensionkultur zu unterschiedlichen Zeitpunkten nach dem Subkultivieren entnommen (Experiment A: 0 d, 50 * 10³ Zellen/ml; B: 2 d, 300 * 10³ Zellen/ml; C: 3 d, 850 * 10³ Zellen/ml) und mittels einer sterilen Kanüle über die Versorgungsleitung in die Zellkammer gefüllt. Die Zellen in der Zellkammer setzten sich nach etwa 10 min auf die Membran ab. Die Reaktionskammer wurde mit einem konstanten Fluss von 75 µl/min (Peristaltikpumpe) mit MS-Medium durchströmt. Alle 10 min wurde aus dem die Stoffkammer verlassenden MS-Medium eine Probe für die NMR-Analytik entnommen. Die Zellen wurden nach 72 bzw. 96 h aus der Zellkammer entnommen und hinsichtlich Vitalität, Zellteilung und Zell-Zell-Kommunikation analysiert (ermittelt mit Standardmethoden [10]).

| Experiment | Beladen: Zellalter (d) | Zeit im mikrofluidischen Bioreaktor (h) | Entnahme: Zellalter (d) |
|---|---|---|---|
| A | 0 | 96 | 4 |
| B | 2 | 72 | 5 |
| C | 3 | 96 | 7 |
| D | Durchschnittswerte der Experimente A-C | | |
| Kontrolle aus Suspensionskultur | | | 4, 5, 7 |

Es konnte gezeigt werden, dass die Zellen, die im mikrofluidischen Bioreaktor kultiviert wurden, die gleichen Eigenschaften hinsichtlich Vitalität, Zellteilung und Zell-Zell-Kommunikation aufwiesen, wie die Kontrollzellen, die unter Standardbedingungen in Suspensionskultur wuchsen. Die in Fig. 6 bis 8 gezeigten Diagramme stellen 3000 Zellen (Vitalität, Mitotischer Index) oder Zellfäden (Häufigkeitsverteilung Zell-Zell-Kommunikation) aus jeweils drei unabhängigen experimentellen Serien dar. Die Fehlerbalken zeigen Standardfehler. Die Überlebensrate 4 (A), 5 (B) bzw. 7 (C) Tage alter Zellen lag sowohl bei den im Bioreaktor kultivierten Zellen als auch bei den Kontrollzellen aus der Suspensionskultur über 95 % (Fig. 6). Die Bestimmung des Mitoseindex (Fig. 7)zeigte, dass die Teilungsrate der Zellen im Bioreaktor vergleichbar mit der der Kontrollzellen war. In der exponentiellen Wachstumsphase (Tag 1 bis 4) lag der Mitoseindex bei beiden Ansätzen zwischen 4 und 6,5 %.

Auch hinsichtlich Zell-Zell-Kommunikation und koordiniertem Wachstum konnten keine Abweichungen zwischen beiden Versuchsansätzen festgestellt werden (Fig. 8). Die charakteristischen Maxima hinsichtlich der Häufigkeitsverteilung von zweizelligen (25 %), vierzelligen (27 %) und sechszelligen Fäden (16 %) der 4 Tage alten Kultur waren bei beiden Versuchsansätzen nachweisbar.

Mittels NMR-Analytik konnten neben der Untersuchung metabolischer Flüsse auch zahlreiche Substanzen, die von den im Bioreaktor kultivierten Zellen in den Mediumfluss abgegeben wurden, in Konzentrationen von 10 µm bis 100 mM nachgewiesen werden (z.B. Glycolsäure, Phosphoethanolamin, Sarkosin, Weinsäure, Taurin, Trimethylaminoxid, Trimethylamin).

### Bezugszeichenliste

- 1: mikrofluidischer Bioreaktor
- 2, 3, 4, 5, 6, X: Modul
- 10: Reaktionsbereich
- 11: Vorratsgefäß
- 12: Sammelgefäß
- 13, 14, 15: Zellen
- 16: Fluidleitungssystem
- 20,: Membran
- 21, 31, 41: erste Einheiten
- 22, 32, 42: zweite Einheiten
- 23, 33, 43, X3: Versorgungsgefäß
- 211, 311, 411: Zellkammer
- 212, 312, 412: Versorgungsleitung
- 213, 313, 413: Abflussleitungen
- 214: Deckel
- 221, 321, 421: Stoffkammern
- 222: Zuflussleitungen
- 223: Verbindungsleitungen

### Literatur

[1] Xia Y., Whitesides G.M.: Soft Lithography. Angew Chem, Int Ed Engl 1998, 37(5):550-575.
[2] Harrison D.J., Manz A., Fan Z., Lüdi H, Widmer H.M.: Capillary Electrophoresis and Sample Injection Systems Integrated on a Planar Glass Chip, Anal. Chem. 1992, 64, S. 1926-1932.
[3] Manz A., Harrison D.J., Verpoorte E.M.J., Fettinger J.C., Paulus A., Lüdi A., Widmer H.M.: Planar chips technology for miniaturization and integration of separation techniques into monitoring systems: Capillary electrophoresis on a chip, J. Chromatogr. 1992, 593, S. 253-258.
[4] Whitesides G.M.: The origins and the future of microfluidics. Nature 2006, 442(7101):368-373.
[5] Ko, J.M., Ju, J., Lee, S., Cha, H. C.: Tobacco protoplast culture in a polydimethylsiloxane-based microfluidic channel. Protoplasma, 2006. 227: S. 237-240.
[6] Thiebaud P., Lauer L., Knoll W., Offenhäusser A.: PDMS device for patterned application of microfluids to neuronal cells arranged by microcontact printing Biosensors and Bioelectronics, 2002. 17: S 87-93.
[7] Murashige T., Skoog F.: A revised medium for rapid growth and bio assays with tobacco tissue cultures. Physiologia Plantarum 1962. 15,3: 473-497; Linsmaier E. M., Skoog F:. Organic growth factor requirements of tobacco tissue cultures. Physiologia Plantarum 1965. 18,1: 100-127; Gamborg O. L., Miller R. A., Ojima K:. Nutrient requirement of suspension cultures of soybean root cells. Exp. Cell Res. 1968. 50,1: 151-158; Smith R. H.: Plant tissue culture, Elsevier, Academic press, 2012
[8] Namdeo A. G.: Plant cell elicitation for production of secondary metabolites: a review. Phcog. Rev. 2007. 1,1: 69-79; Santner A., Calderon-Villalobos L. I. A., Estelle M.: Plant hormones are versatile chemical regulators of plant growth. Nature Chemical Biology 2009. 5,5: 301-307; Qiao F., Petrasek J., Nick P.: Light can rescue auxin-dependent synchrony of cell division in a tobacco cell line. J. Exp. Botany 2010. 61,2: 503-510; Hicks G. R., Raikhel N. V.: Small molecules present large opportunities in plant biology. Annu. Rev. Plant Biol. 2012. 63: 261-282.
[9] Maisch J., Nick P.: Actin is involved in auxin-dependent patterning. Plant Physiol. 2007. 143: 1659-1704.
[10] Campanoni P., Blasius B., Nick P.: Auxin transport synchronizes the pattern of cell division in a tobacco cell line. Plant Physiol. 2003. 133: 1251-1260.
[11] Desikan R., Hancock J. T., Coffey M. J., Neill S. J.: Generation of active oxygen in elicited cells of Arabidopsis thaliana is mediated by a NADPH oxidase-like enzyme. FEBS letters 1996. 382: 213-217.
[12] Cao J., Duan X., McElroy D., Wu R.: Regeneration of herbicide resistant transgenic rice plants following microprojectilemediated transformation of suspension culture cells. Plant Cell Reports 1992. 11: 586-591.
[13] Yeoman M. M., Yeoman C. L.: Manipulating secondary metabolism in cultured plant cells. New Phytol. 1996. 134: 553-569.

## Patentansprüche

1. Verfahren zur Gewinnung von Zellmetaboliten mit einem mikrofluidischen Bioreaktor (1) umfassend
- mindestens zwei Module (2, 3, 4, 5, 6, X) mit jeweils einer Kavität, die mit jeweils einer Membran (20) in jeweils eine Zellkammer (211, 311, 411) für die Aufnahmen von Zellen und jeweils einer mit einer flüssigen Lösung mit mindestens einem Zusatzstoff und Zellmetaboliten durchströmbare Stoffkammer (221, 321, 421) unterteilt ist, wobei die jeweilige Membran einen Reaktionsbereich (10) aufweist, in dem die Membran (20) zumindest teilweise durchlässig für die Lösung mit dem mindestens einem Zusatzstoff und die Zellmetaboliten ist, sowie
- ein Fluidleitungssystem (16) für die flüssige Lösung mit dem mindestens einem Zusatzstoff und die Zellmetaboliten, das die Stoffkammer (221, 321, 421) in Reihe und/oder parallel miteinander verbindet
- sowie ein Mittel für eine unidirektionale Durchströmbarkeit des Fluidleitungssystem.
mit den Verfahrensschritten:
a) Einbringen von Zellen in die Zellkammer (211, 311, 411)
b) Anlegen eines Flüssigkeitsstroms einer flüssigen Lösung mit mindestens einem Zusatzstoff im Fluidleitungssystem des mikrofluidischen Bioreaktors (1) zur Synthese des mindestens einen Zellmetaboliten, wobei die flüssige Lösung mit dem mindestens einem Zusatzstoff mindestens zwei Module nacheinander durchfliest und dabei jeweils über die Stoffkammern (221, 321, 421) durch die Membran in die Zellkammer (211, 311, 411) eindringt, in der Zellkammer (211, 311, 411) mit den Zellen mindestens ein Produkt synthetisiert und die flüssige Lösung mit dem mindestens einem Zusatzstoff und dem mindestens einem Produkt durch die Membran über die Stoffkammern (221, 321, 421) in das Fluidleitungssystem (16) zurückgeführt wird, wobei das Produkt des zuvor durchflossenen Moduls das Edukt des nachfolgenden Moduls ist, bis das Produkt der Zellmetabolit ist
c) Entnahme des mindestens einen Zellmetaboliten aus dem Flüssigkeitsstrom.

2. Verfahren zur Gewinnung von Zellmetaboliten mit einem mikrofluidischen Bioreaktor (1) nach Anspruch 1, **dadurch gekennzeichnet, dass** im Verfahrensschritt a) in die jeweiligen Zellkammern (211, 311, 411) der Module (2, 3, 4, 5, 6, X) die gleiche Zelllinie oder verschiedene Zelllinien oder teilweise verschiedene Zelllinien eingebracht werden.

3. Verfahren zur Gewinnung von Zellmetaboliten mit einem mikrofluidischen Bioreaktor (1) nach Anspruch 1 oder 2, **dadurch gekennzeichnet, dass** im Verfahrensschritt a) die Einbringung der Zellen in einer Nährlösung über die Versorgungsleitung (212, 312, 412) erfolgt und überschüssiges Volumen der Nährlösung durch die Abflussleitung (213, 313, 413) entfernt wird.

4. Verfahren zur Gewinnung von Zellmetaboliten mit einem mikrofluidischen Bioreaktor (1) nach einem der Ansprüche 1 bis 3, **dadurch gekennzeichnet, dass** die Synthese im Verfahrensschritt b) durch Zugabe mindestens eines Aktivators zu mindestens einer Zelllinie in mindestens einer Zellkammer (211, 311, 411) ausgelöst wird.

## Claims

1. Method for obtaining cell metabolites with a microfluidic bioreactor (1) consisting of
- at least two modules (2, 3, 4, 5, 6, X) each having a cavity that is subdivided by a membrane (20) into a cell chamber (211, 311, 411) for receiving cells and a material chamber (221, 321, 421) through which a liquid solution that has at least one additive and cell metabolites can flow, with each membrane comprising a reaction region (10) in which the membrane (20) is at least partly permeable for the solution that has at least one additive and the cell metabolites, and
- a fluid conducting system (16) for the liquid solution that has the at least one additive and the cell metabolites, and which connects said material chamber (221, 321, 421) in series and/or parallel with one another
- and means for unidirectional flow through the fluid conducting system.
with the method steps:
a) introducing cells into the cell chamber (211, 311, 411)
b) creating a liquid flow of a liquid solution that has at least one additive in the fluid conducting system of the microfluidic bioreactor (1) for the synthesis of the at least one cell metabolite, with the liquid solution that has the at least one additive flowing through at least two modules in succession and in the process penetrating the cell chamber (211, 311, 411) via the material chambers (221, 321, 421) through the membrane, then in the cell chamber (211, 311, 411) synthesising at least one product with the cells, and with the liquid solution that has the at least one additive and the at least one product being returned through the membrane via the material chambers (221, 321, 421) into the fluid conducting system (16), with the product of the module through which the solution previously flowed being the educt of the subsequent module, until the product is the cell metabolite
c) removing the at least one cell metabolite from the liquid flow.

2. Method for obtaining cell metabolites with a microfluidic bioreactor (1) according to claim 1, **characterised in that** in method step a), the same cell line or different cell lines or partially different cell lines are introduced into the respective cell chambers (211, 311, 411) of the modules (2, 3, 4, 5, 6, X).

3. Method for obtaining cell metabolites with a microfluidic bioreactor (1) according to claim 1 or 2, **characterised in that** in method step a) the introducing of the cells in a nutrient solution is effected through the supply line (212, 312, 412) and surplus volume of the nutrient solution is removed through the discharge line (213, 313, 413).

4. Method for obtaining cell metabolites with a microfluidic bioreactor (1) according to any one of claims 1 to 3, **characterised in that** the synthesis in method step b) is initiated by the addition of at least one activator to at least one cell line in at least one cell chamber (211, 311, 411).

## Revendications

1. Procédé permettant d'obtenir des métabolites cellulaires avec un bioréacteur microfluidique (1) comprenant :
- au moins deux modules (2, 3, 4, 5, 6, X) ayant chacun une cavité qui est subdivisée par une membrane respective (20) en une chambre cellulaire respective (211, 311, 411) destinée à la réception de cellules, et une chambre de produits respective (221, 321, 421) dans laquelle peut circuler une solution liquide renfermant au moins un additif et des métabolites cellulaires,
chacune des membranes (20) comprenant une zone réactionnelle (10) dans laquelle elle est au moins partiellement perméable à la solution renfermant l'additif et les métabolites cellulaires, et
- un système de conduites fluidiques (16) destiné à la circulation de la solution liquide renfermant l'additif et les métabolites cellulaires reliant les chambres de produits (221, 321, 421) en série et/ou en parallèle, ainsi que
- des moyens permettant une circulation unidirectionnelle dans le système de conduites fluidiques,
procédé comprenant des étapes consistant à :
a) introduire des cellules dans la chambre cellulaire (211, 311, 411),
b) établir une circulation d'une solution liquide renfermant au moins un additif dans le système de conduites fluidiques du bioréacteur microfluidique (1) pour permettre la synthèse des métabolites cellulaires, la solution liquide renfermant l'additif traversant successivement au moins deux modules et pénétrant respectivement par les chambres de produits (221, 321, 421), au travers de la membrane, dans la chambre cellulaire (211, 311, 411), dans la chambre cellulaire (211, 311, 411), renfermant les cellules au moins un produit étant synthétisé, et la solution liquide renfermant l'additif et le produit étant renvoyée dans le système de conduites fluidiques (16) au travers de la membrane par l'intermédiaire des chambres de produits (221, 321, 421), le produit du module traversé précédemment constituant le produit de départ du module suivant, jusqu'à ce que le produit soit le métabolite cellulaire,
c) prélèvement du métabolite cellulaire du flux de liquide.

2. Procédé permettant d'obtenir des métabolites cellulaires avec un bioréacteur microfluidique (1), conforme à la revendication 1,
**caractérisé en ce que**
lors de l'étape a), des lignées cellulaires identiques ou des lignées cellulaires différentes ou des lignées cellulaires partiellement différentes sont introduites, dans les chambres cellulaires respectives (211, 311, 411) des modules (2, 3, 4, 5, 6, X).

3. Procédé permettant d'obtenir des métabolites cellulaires avec un bioréacteur microfluidique (1) conforme à la revendication 1 ou 2,
**caractérisé en ce que**
lors de l'étape a) l'introduction des cellules est effectuée dans une solution nutritionnelle par une conduite d'alimentation (212, 312, 412) et le volume de solution nutritionnelle en excès est extrait par une conduite d'évacuation (213, 313, 413).

4. Procédé permettant d'obtenir des métabolites cellulaires avec un bioréacteur microfluidique (1) conforme à l'une des revendications 1 à 3,
**caractérisé en ce que**
lors de l'étape b) la synthèse est déclenchée par l'addition d'au moins un agent d'activation à au moins une lignée cellulaire dans au moins une chambre cellulaire (211, 311, 411).
